# EUROPEAN PATENT SPECIFICATION

(11) **EP 4 313 227 B1**
(45) Date of publication and mention of the grant of the patent: **28.01.2026**
(21) Application number: 22778205.9
(22) Date of filing: 25.03.2022
(51) Int. Cl.: A61M 16/00, A61M 16/06, A61B 5/08, A61B 5/087, A61H 31/00, G09B 23/28, G09B 23/30, G16H 40/63, A61B 5/00, A61M 16/20, G16H 20/40

(54) **VENTILATION ASSISTANCE**
LÜFTUNGSUNTERSTÜTZUNG
DISPOSITIF D'ASSISTANCE AUX VENTILATIONS

(30) Priority: 29.03.2021 AU 2021900924
(43) Date of publication of application: 07.02.2024
(73) Proprietor: The University Of Sydney, Sydney, New South Wales 2006 (AU); Western Sydney Local Health District, Westmead NSW 2145 (AU)
(72) Inventor: CROTT, Matthew, Killarney Heights, NSW 2087 (AU); TRACY, Mark, Glenbrook, NSW 2773 (AU); HINDER, Murray, Leura, NSW 2780 (AU); BOUSTRED, Matthew, Mosman, NSW 2088 (AU)
(74) Representative: Viering, Jentschura & Partner mbB Patent- und Rechtsanwälte
(86) International application number: PCT/AU2022/050272
(87) International publication number: WO 2022/204750

(56) References cited:
- DE-A1- 102005 060 844
- JP-A- 2017 055 965
- US-A1- 2018 021 533
- US-A1- 2018 147 375
- US-A1- 2018 333 548
- US-A1- 2019 070 374
- US-A1- 2019 351 165
- US-A1- 2020 114 102
- US-A1- 2020 345 962

## Description

### CROSS-REFERENCE TO RELATED APPLICATION

This application claims the benefit of priority of Australian Provisional Patent Application No. 2021900924 filed on March 29, 2021.

### FIELD OF THE INVENTION

The present invention relates to the field of ventilation assistance and in particular to a ventilation assistance device and a method of operating such a ventilation assistance device. In particular, such ventilation assistance device may be used for resuscitation and resuscitation training, including resuscitation of infants and in training operators in infant resuscitation.

### BACKGROUND OF THE INVENTION

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

Methods of ventilation assistance, including for resuscitating infants, are known. These methods typically involve an operator operating a resuscitation device to deliver gas such as air and/or oxygen to the airway of the infant. Self-inflating bags and ventilation machines are examples of known resuscitation devices.

Self-inflating bag resuscitators ('bag-mask devices') require an operator to manually compress a self-inflating bag to deliver a volume of gas to the airway of the infant. After the volume of gas has been delivered to the infant, the operator allows the self-inflating bag to self-inflate by releasing pressure on the self-inflating bag. Subsequently, the operator can compress the self-inflating bag again to deliver another volume of gas to the airway of the infant.

Ventilation machines are machines that are able to cyclically deliver a volume of gas to the airway of the infant. Ventilation machines typically provide several controls that an operator can adjust to control one or more characteristics of the volume of gas delivered to the airway of the infant.

For infant resuscitation, babies come in different shapes and sizes. As a result they each have different requirements. Particularly for manual ventilation, but also relevant for machine-assisted ventilation, one of the most important and most variable needs is the amount of gas being delivered to the baby's lungs. A small baby needs a small amount of air and a large baby needs a larger amount of air. Too much gas and the baby's lungs can be damaged through overdistension, or oxygen flow restricted to the brain, leading to serious complications such as brain or lung damage. Conversely, too little gas, resulting in a lack of oxygen for the infant, has the potential to lead to brain damage or even death. This concept of ensuring the volume of air is correct for the size of the infant is often not taught (or not taught sufficiently clearly) during newborn resuscitation training, where trainees are generally trained just to observe the baby's chest rising and falling with ventilation. While respiratory function monitors are able to monitor airflow and display information on tidal volumes in millilitres, airway pressures or other unit, operators without advanced training are unable to correctly interpret such readouts, particularly in resuscitation situations, and such errors in interpretation could lead to disastrous consequences. This precludes the use of respiratory function monitors in neonatal resuscitation by operators without advanced training.

JP 2017 055965 A describes a breathing state display device and method, including calculation of a first index indicating a normal or abnormal respiration parameter based on acquired body index.

US 2020/114102 A1 describes a medical ventilation apparatus arranged to supply a gas circuit with respiratory gas and allowing selection from a stored plurality of ventilation modes and a plurality of patient category selectors.

DE 10 2005 060844 A1 described a method and a device involving using a control mechanism to manually select between stored artificial respiration parameters, the parameters visualisable on a display.

US 2018/147375 A1 describes resuscitation and ventilation monitoring device are provided with an inlet in fluid communication with airflows exchanged with lungs of a patient and an airflow meter measuring characteristics of the airflows.

US 2018/333548 A1 describes a system adapted to be attached to a human patient or a mannequin, with an output unit connected to the processor, and configured to output at least one of a condition of the mask adapted to be attached to the human patient or the mannequin; and a condition of the human patient or the mannequin.

US 2015/0096559 A1 describes manual ventilation feedback sensor for use in clinical and training settings with a sensing module with a controller for processing information from a pressure sensor and/or flow transducer for determining and indicating a ventilation rate.

DE 10 2008 028662 describes a device for measuring and signaling at least one ventilation parameter and / or a physiological parameter, which is detachably arranged in a region of a respiration device formed without a signaling- and measuring device.

Embodiments of the present invention seek to address at least one of the above problems.

### SUMMARY OF THE INVENTION

According to a first aspect, the present invention provides a ventilation assistance device as defined in claim 1.

According to a second aspect, the present invention provides a method of using a ventilation assistance device for providing ventilation to a subject, as defined in claim 8.

Additional features of example embodiments are defined in the dependent claims.

### BRIEF DESCRIPTION OF THE DRAWINGS

Preferred embodiments of the present invention will be described, by way of examples only, with reference to the accompanying figures, wherein:
Figure 1 shows a ventilation assistance device according to an embodiment of the present invention;
Figure 2(A) shows a sample display of a ventilation assistance device according to an example embodiment;
Figure 2(B) shows a sample display of a ventilation assistance device according to an example embodiment;
Figure 2(C) shows a sample display of a ventilation assistance device according to an example embodiment;
Figure 2(D) shows a sample display of a ventilation assistance device according to an example embodiment;
Figure 2(E) shows a sample display of a ventilation assistance device according to an example embodiment;
Figure 2(F) shows a sample display of a ventilation assistance device according to an example embodiment;
Figure 3 shows a patient ventilation set-up incorporating the ventilation assistance device of Figure 1;
Figure 4 shows a schematic diagram illustrating a ventilation assistance device according to an example embodiment; and
Figure 5 shows a flowchart illustrating a method for ventilation assistance according to an example embodiment.

### DETAILED DESCRIPTION OF THE EMBODIMENTS

An example embodiment of the present invention provides the user with quantitative data relating to ventilation assistance that is easy to interpret and intuitive to use. A 'symbolic logic' of the feedback-based solution according to an example embodiment of the invention allows users to adjust, optimize and improve their technique during actual ventilation assistance and during training, so that they are optimally equipped for action during manual ventilation, in particular in babies following birth.

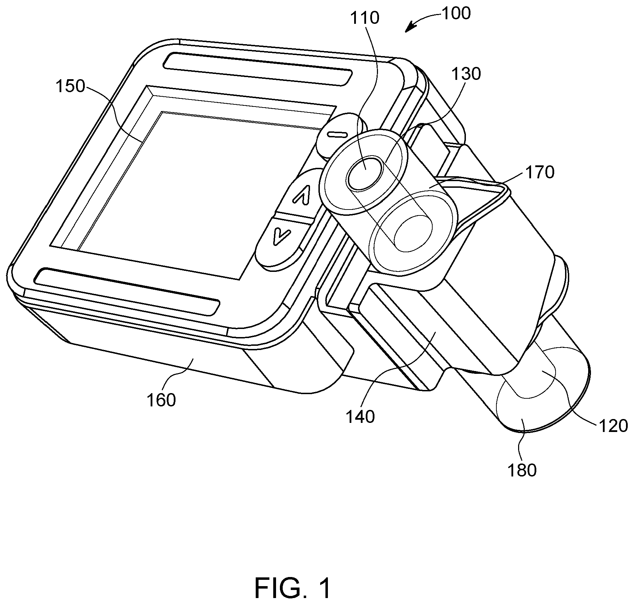

Figure 1 shows a ventilation assistance device 100 according to an example embodiment of the present invention. The ventilation assistance device 100 has a duct in the form of a tube 130 with a first opening 110 at a first end, a second opening 120 at a second end, as well as a flow sensor 140 and associated control circuity inside the housing 160 of the ventilation assistance device 100, and an operator interface in the form of a display 150 and associated control circuity inside the housing 160 of the ventilation assistance device 100. It will be appreciated that the example shape and configuration of the ventilation assistance device and its components are not limited to the example shape and configuration as shown for the example embodiment in Figure 1. It will further be appreciated that the circuitry for the display 150 and the flow sensor 140 may be separately implemented, or may be implemented in one processing circuit. It will further be appreciated that the circuitry may be integrally provided in the housing 160 circuit and/or may be externally provided with suitable interconnection media.

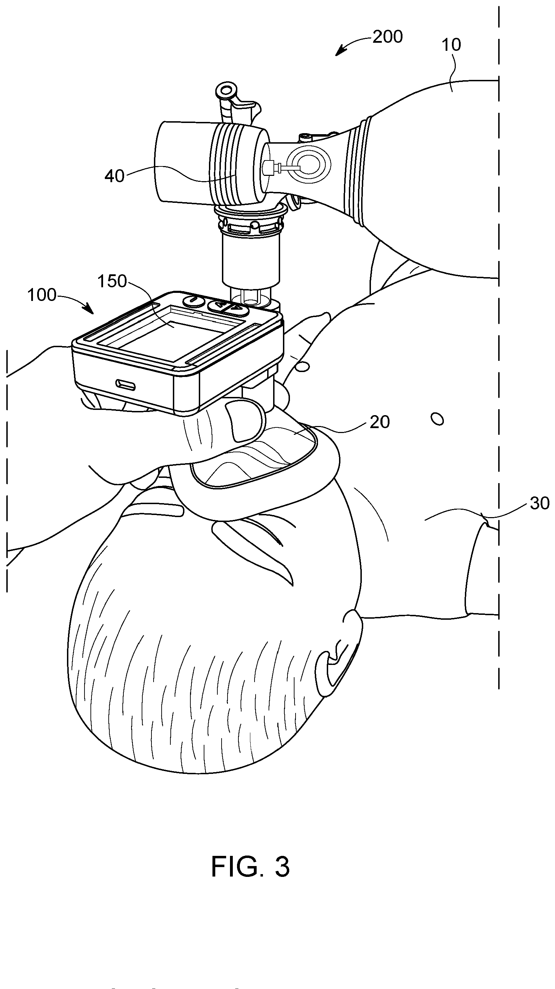

The first opening 110 is configured to be coupled in fluid communication with a source of gas (generally, air and/or oxygen) 10 (see Figure 3). In Figure 3, the gas source 10 is illustrated as a self-inflating bag, however, the gas source 10 may be a ventilator, anaesthetic bag, T- piece resuscitator, or any other suitable device as understood in the art for supplying gas for use in resuscitation or continuous positive airway pressure (CPAP) support. The source of gas 10 will be referred to below as "gas source 10" for convenience. With reference again to Figure 1, the first opening 110 may be coupled to an outlet of the gas source 10 by a friction fit, respective complementary threads, a Luer fitting, or any other suitable means capable of coupling the first opening 110 in fluid communication with the gas source 10. In the embodiment shown in Figure 1, an outer tube portion 170 is provided around the first opening 110 for friction fit with a tube-shaped coupler on the gas source 10.

The second opening 120 is configured to be coupled in fluid communication with an interface device 20 (see Figure 3). The interface device 20 may be a face mask, endotracheal tube, laryngeal mask airway, nasal cannulae, or any other suitable device as understood in the art that is capable of delivering gas to the airway of a subject 30. The interface device will be referred to below as "interface device 20" for convenience. The second opening 120 may be coupled to the interface device 20 by any suitable means, as discussed above with reference to the coupling between first opening 110 and gas source 10, applied to the second opening 120 and/or the second end 125. In the embodiment shown in Figure 1, an outer tube portion 180 is provided around the first opening 120 for friction fit with a tube-shaped coupler on the interface device 20.

Referring still to Figure 1, the tube 130 extends between the first opening 110 and the second opening 120 to provide a fluid connection therebetween. In other words, when the first opening 110 is coupled in fluid communication with a gas source 10 and the second opening 120 is coupled in fluid communication with an interface device 20, and the gas source 10 and the interface device 20 are in fluid communication with each other via the tube130.

The flow sensor 140 is configured to measure the rate of a gas flow through the tube 130. The flow sensor 140 may be a single sensor configured to measure flow through the tube 130 from the second opening 120 to the first opening 110 and to measure flow through the tube 130 from the first opening 110 to the second opening 120, i.e. bi-directionally. Alternatively, the flow sensor 140 may comprise two uni-directional flow sensors, one configured to measure flow through the tube 130 from the first opening 110 to the second opening 120 and another flow sensor to measure flow through the tube 130 from the second opening 120 to the first opening 110. The flow measured may be sensed as a flow rate (eg. in L/min) or as a flow volume (eg. mL supplied or returned in a single ventilatory cycle), preferably for a single ventilation cycle.

The flow sensor 140 may be any suitable single sensor or two sensors as understood in the art capable of measuring flow through the tube 130. This includes through a hot-wire anemometer, differential pressure pneumotach, micro-electromechanical systems (MEMS) sensors, such as thermal MEMS sensors or MEMS piezoelectric sensors, or any other suitable flow sensing method as understood in the art. Depending on the type of sensor(s) used, the flow sensor 140 may be positioned within the tube 130 (e.g. hot-wire anemometers and MEMS flow sensors) or external to the tube 130 (e.g. ultrasonic flow meters) inside the housing 160. In the example embodiment, the flow sensor comprises a thermal MEMS sensor positioned internal to the tube 130.

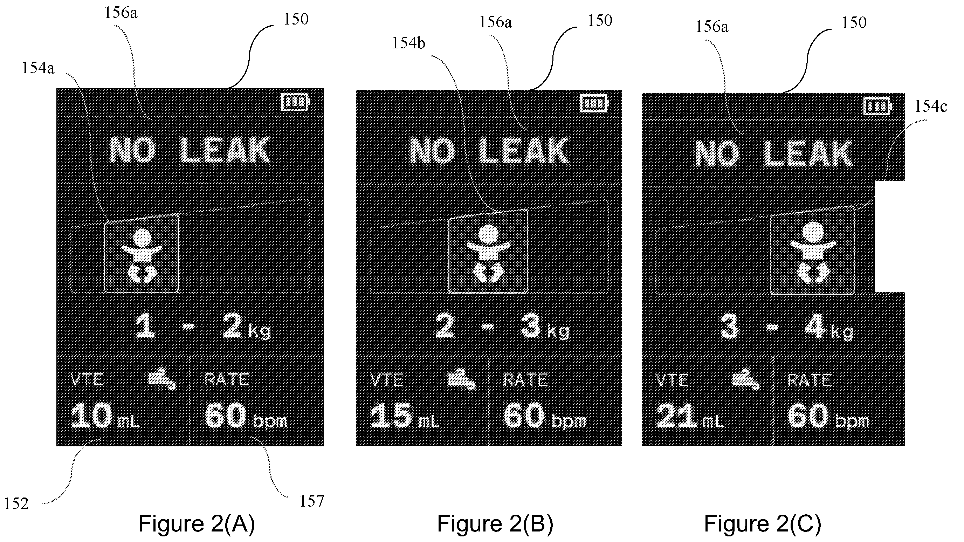

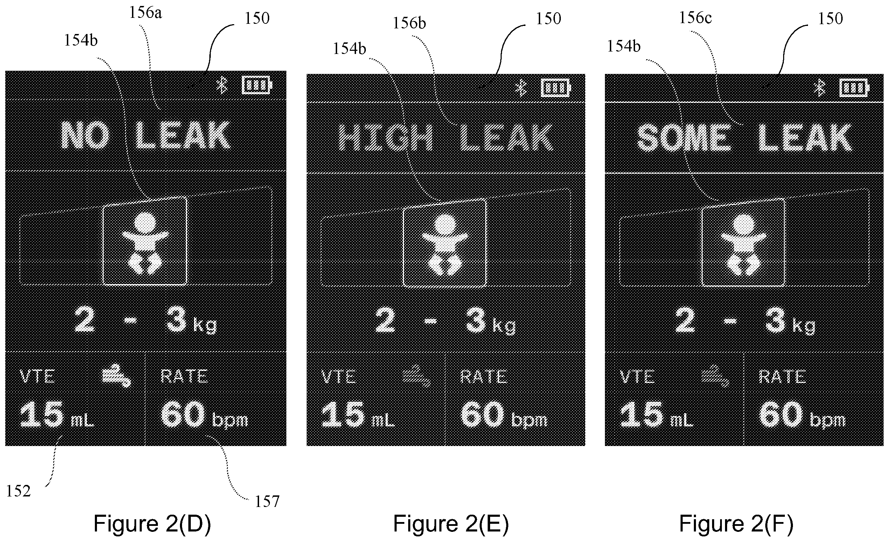

Figures 2(A) to (F) show example screen shots of the display 150 according to an example embodiment of the invention. The display 150 has a ventilatory volume indicator 152, infant size indicators 154, leakage indicators 156, and ventilatory rate indicator 157. The ventilatory volume indicator 152, infant size indicators 154, and leakage indicators 156a-c represent characteristics of the flow of gas through the tube 130 for each ventilation cycle, as will be discussed in detail below.

In use, the first opening 110 and the second opening 120 of the ventilation assistance device 100 are coupled in fluid communication with the gas source 10 and a interface device 20, respectively, and the interface device 20 is positioned to deliver gas to the airway of a subject 30. For example, if the interface device 20 is a resuscitation mask (see Figure 3), the resuscitation mask is placed over the nose and mouth of the subject 30. Together, the air source 10, the ventilation assistance device 100, and the interface device 20 may form a patient ventilation set-up 200 (see Figure 3).

With reference now to Figure 3, the patient ventilation set-up 200 is being used to cyclically supply gas to the subject 30 and expel air subsequently exhaled by the subject 30. Each cycle of supplying gas to the subject 30 and expelling air subsequently exhaled by the subject 30 is referred to herein as a ventilation cycle. Accordingly, each ventilation cycle comprises supplying gas to the subject 30, which is at least partially inhaled by the subject 30, and expelling at least some of the gas subsequently exhaled by the subject 30.

For each ventilation cycle, an operator operates the gas source 10 to supply gas (i.e. a inspiratory volume) to the subject 30 and expel at least some of a volume of gas that is subsequently exhaled by the subject 30 (i.e. the expiratory volume). For each ventilation cycle, the inspiratory volume flows from the air source 10, through the tube 130 of the ventilation assistance device 100, to the interface device 20, where the inspiratory volume is at least partially inhaled by the subject 30. At least some of the inhaled gas that is subsequently exhaled by the subject 30 (i.e. the expiratory volume) then flows into the interface device 20, through the tube 130 of the ventilation assistance device 100, and is expelled to atmosphere (e.g. via a one-way valve 40 in the gas source 10).

As an example, the gas source 10 may be a manual resuscitator comprising a self-inflating bag as shown in Figure 3 (a 'bag-valve mask device'). The operator operates such a device by compressing the self-inflating bag to partially deflate it, causing an inspiratory volume to be delivered from the air source 10 to the airway of the subject 30 via the ventilation assistance device 100 and the interface device 20. The operator then releases the self-inflating bag to allow it to reinflate, before compressing it once again to deliver another inspiratory volume to the subject 30. The frequency of the ventilation cycle is dependent on how often an operator compresses the self-inflating bag in a set time period. The inspiratory volume supplied to the airway of the subject 30 for each ventilation cycle is dependent on the amount of compression the operator applies to the self-inflating bag.

As another example, if the air source 10 is a flow-driven resuscitation device (a 'flow-driven device'), the operator may operate the flow-driven device by adjusting one or more device controls, including the peak inspiratory pressure (PIP) and the positive end expiratory pressure (PEEP). The operator can then deliver each inspiratory volume by occluding a one-way valve with their finger to redirect the air through the ventilation assistance device 100 and the interface device 20, and can vary the frequency of ventilatory cycles by varying the frequency of these occlusions.

For each ventilatory cycle, the ventilation assistance device 100 in this example embodiment uses the flow sensor 140 to determine the expiratory volume of gas and the inspiratory volume of gas that flow through the tube 130 subject 30. For example, and with reference again to Figures 2(A) to (F), the calculated expiratory volume is displayed at the ventilatory volume indicator 152 (e.g. in mL) on the display 150.

Referring still to Figures 2(A) to (F), the infant size indicators 154a-c in the example embodiment include three infant size indicators 154a-c representing images of differently sized infants. Each infant size indicator 154a-c represents an infant weight range (e.g. in kilograms). As an example, infant size indicator 154a may represent an infant weight range between 0.5 to 1.4 kg, infant size indicator 154b may represent an infant weight range between 1.5 to 3.4 kg, and infant size indicator 154c may represent an infant weight range between 3.5 to 5 kg. Although Figures 2(A) to (F) only illustrate three infant size indicators 154a-c, it will be appreciated that more generally two or more infant size indicators may be provided, each representing a predefined infant weight range. It will also be appreciated that indicators of infant gestational age could be substituted for indicators of infant weight range as another characteristic of the infant.

For each ventilation cycle, the ventilation assistance device 100 is configured to display or highlight on the display 150 one of the infant size indicators 154a-c to indicate an infant weight range that is suitable for the calculated expiratory volume. In the example of Figures 2(B) and (D) to (F), for a given ventilation cycle, the ventilatory volume indicator 152 indicates a calculated expiratory volume of 15 mL and the infant size indicator 154b is highlighted, indicating that this expiratory volume is suitable for mid-size infants (e.g. infants weighing between 2 to 3 kg). As an example, the ventilation assistance device 100 may display or highlight on the display 150 the:
Infant size indicator 154a for expiratory volumes between 5mL to 12mL for 1-2kg infants;
Infant size indicator 154b for expiratory volumes between 12mL to 18mL for 2-3kg infants; and
Infant size indicator 154c for expiratory volumes between 18mL to 24mL for 3-4kg infants.

In the example of Figures 2(A) to (F), infant size indicator 154b is highlighted by showing an image representing the infant size, while the other infant size indicators 155a, c are blank. Alternatively, the display 150 may be configured to display on the display 150 images representing different infant sizes and highlight e.g. by changing from outline to solid filling, the one indicator 154a-c corresponding to the calculated expiratory volume. It is also envisaged that the infant size indicators 154a-c may be displayed or highlighted using any other suitable methods as understood in the art (e.g. visual, audible, and/or haptic indications).

Expiratory volume is related to the tidal volume of the infant, which varies depending on the infant's size or gestational age. Due to this association, a larger expiratory volume is generally appropriate for a larger infant size, and a smaller expiratory volume is generally appropriate for a smaller infant size. Inspiratory volume that is measured before the interface device 20 might not correspond to tidal volumes if there is a significant leak around the interface device 20 that results in some or all of the gas being vented to atmosphere rather than being delivered to the infant.

If the interface device 20 is not correctly positioned, a substantial amount of the inspiratory volume may not be inhaled by the subject 30 and may escape (i.e. leak) to atmosphere. This may result in less than the required volume of gas being inhaled by the subject 30 for each ventilation cycle. As an example, if the interface device 20 is a resuscitation mask and it is not correctly positioned over the nose and mouth of the subject 30, some of the inspiratory volume may escape between the interface between the resuscitation mask and the subject 30's face.

For each ventilation cycle, the ventilation assistance device 100 is configured to display or highlight on the display 150 one of the leakage indicators 156a-c to indicate the amount of leakage occurring during each ventilation cycle. Leakage is determined based on the inspiratory volume and expiratory volume. It is calculated as the difference between the inspiratory volume and expiratory volume as a percentage of the inspiratory volume. As an example, an inspiratory volume of 10mL and expiratory volume of 5mL would give a leak of 50%.

In the example of Figures 2(A) to (F), the ventilation assistance device 100 may be configured to display or highlight on the display 150 the:
leakage indicator 156b if the ventilation assistance device 100 calculates that more than 60% of the inspiratory volume has been leaked;
leakage indicator 156c if the ventilation assistance device 100 calculates that between 30 - 60% of the inspiratory volume has been leaked; and
leakage indicator 156a if the ventilation assistance device 100 calculates that between 0 - 30% of the inspiratory volume has been leaked.

The leakage indicators 156a-c may indicate the amount of leakage using visual indications. As an example, the leakage indicators 156a-c may use words and font color (based on traffic light), as shown in Figures 2(A) to (F):
leakage indicator 156b may display "high leak" in red font color;
leakage indicator 156c may display "some leak" in yellow/amber font color; and
leakage indicator 156a may display "no leak" in green font color.

Alternatively, other visual, audible, and/or haptic indications may be used, such as, but not limited to, a traffic light system based only on color.

Based on the leakage indicator 156a-c displayed on the display 150, an operator may be able to adjust the interface device 20 and/or any other aspect of the patient ventilation set-up 200 (see Figure 3) to reduce the amount of leakage per ventilation cycle. Reducing the amount of leakage may increase the likelihood of the correct volume of gas being inhaled by the subject 30 during each ventilation cycle.

The display 150 may be configured to only display the leakage indicator 156a-c corresponding to the calculated leakage as shown in Figures 2(A) to (F). Alternatively, display 150 may be configured to highlight display all the leakage indicators 156a-c and to highlight the one corresponding to the calculated leakage, e.g. using a higher intensity and/or by changing from outline to solid filling. It is also envisaged that the leakage indicators 156a-c may be displayed or highlighted using any other suitable methods as understood in the art (e.g. visual, audible, and/or haptic indications). Although Figures 2(A) to (F) illustrates three leakage indicators 156a-c, it will be appreciated that more or less leakage indicators may be provided, each representing a predefined amount of leakage (e.g. in percentage of the inspiratory volume). In the example embodiment, three different leakage indicators 156a-c are provided, however the number can be different in different embodiments, including only one indicator for indicating when the leak is low.

Ventilatory rate is also calculated and displayed, see ventilatory rate indicator 157. It is determined by measuring the frequency of the ventilations by detecting the beginning of each inspiration, and then then displaying the rate per minute.

The "subject" discussed above may be an infant or a manikin of an infant, as shown in Figure 3. Accordingly, the flow monitor 100 may be used to assist with resuscitating an infant weighing within a particular weight range and/or to train operators to resuscitate infants falling within different weight ranges. If the subject 30 is a manikin of an infant, the manikin may include a face and an airway, and be designed to provide observable chest movement to simulate lung inflation and deflation of an infant during resuscitation. Accordingly, the manikin may be used to replicate ventilation of an infant. There are different manikin sizes available in the market to represent different age/weight/size infants. The "matching" of the suitable size indicator according to example embodiments can either be done through the given specifications (e.g. the manufacturer specifies that this is a manikin that represents a 3kg baby) or by simulation (e.g. the trainer says 'pretend this is a 2kg baby', as non-limiting examples.

It is noted that the present invention is not limited to being applied to infants or manikins of infants, but can be applied generally to humans, including to other target groups, including to older pediatric patients and adults, or manikins thereof.

Use of the ventilation assistance device 100 to resuscitate an infant as a non-limiting example will now be described.

The first opening 110 and the second opening 120 of the ventilation assistance device 100 are coupled in fluid communication with a gas source 10 and a interface device 20, respectively, as described above. The ventilation assistance device 100, gas source 10, and interface device 20 together may form a patient ventilation set-up 200, as shown in Figure 3.

Subsequently, an operator will position the interface device 20 to deliver gas to the airway of the infant. As an example, if the interface device 20 is a resuscitation mask, the interface device 20 is placed over the nose and mouth of the infant. As another example, if the interface device 20 is an endotracheal tube or a laryngeal mask airway, the interface device 20 is partially inserted into the airway of the infant.

The operator operates the gas source 10 as described above to supply a volume of gas (i.e. a inspiratory volume) to the infant and to expel at least some of the volume of gas subsequently exhaled by the infant (i.e. the expiratory volume) for each ventilation cycle.

With reference to Figures 2(A) to (F), for each ventilation cycle, the ventilation assistance device 100 in the example embodiment uses the flow sensor 140 to calculate the expiratory volume, inspiratory volume, leakage, and ventilatory rate, as described above. Based on the calculated expiratory volume and leakage, the ventilation assistance device 100 displays on the display 150 the infant size indicator 154a-c and the leakage indicator 156a-c corresponding to the calculated expiratory volume and leakage, respectively. The ventilation assistance device 100 may also display on the display 150 the calculated inspiratory volume at the ventilatory volume indicator 152, for example alternatingly with the expiratory, or based on user control of the display 150.

The infant the operator is resuscitating will have particular physiological characteristics (e.g. weight, size and gestational age). Infants of different weight/size and/or gestational age will require to receive different gas volumes to be delivered to their airway during resuscitation. Receiving a gas volume that is too large for a particular infant may result in damage to the infant's lungs or brain, and receiving a gas volume that is less than a required volume may cause brain or organ damage to the infant or result in the death of the infant through a lack of oxygen. Accordingly, it is desirable to maintain the gas volume received by the infant for each ventilation cycle within a particular range depending on the weight/size of the infant.

Based on the infant size indicator 154a-c displayed on the display 150, the operator is able to observe if the gas volume received by the infant for a ventilation cycle is appropriate for the infant they are resuscitating. If the infant size indicator 154a-c displayed on the display 150 for a ventilation cycle corresponds to the weight/size of the infant being resuscitated, the operator continues to operate the resuscitation device source 10 using the current mode of operation. As an example, if the gas source 10 is a self-inflating bag, the operator will continue compressing the self-inflating bag to a similar degree for the next ventilation cycle so that a similar gas volume is received by the infant. As another example, if the gas source 10 is a flow-driven device, the operator does not adjust the control settings of the flow-driven device so that the same gas volume is received by the infant for the next ventilation cycle.

If the infant size indicator 154a-c displayed on the display 150 does not correspond to the weight/size of the infant being resuscitated, the operator will adjust their mode of operating the gas source 10 so that the gas volume received by the infant for subsequent ventilation cycles is appropriate for the infant being resuscitated.

For example, if the infant size indicator 154a-c displayed on the display 150 indicates that the calculated inspiratory volume corresponds to an infant weight range that is less than the infant being resuscitated, the operator will adjust their mode of operating the gas source 10 to increase the inspiratory volume delivered to the infant for the next ventilation cycle. As an example, if the gas source 10 is a self-inflating bag, the operator will increase the degree of compression of the self-inflating bag for the next ventilation cycle to increase the inspiratory volume delivered to the infant. As another example, if the gas source 10 is a flow-driven device, the operator may adjust the peak inspiratory pressure of the flow-driven device to increase the inspiratory volume delivered to the infant for the next ventilation cycle.

As another example, if the infant size indicator 154a-c displayed on the display 150 indicates that the calculated inspiratory volume corresponds to an infant weight range that is greater than the weight range of the infant being resuscitated, the operator will adjust their mode of operating the gas source 10 to decrease the inspiratory volume delivered to the infant for the next ventilation cycle. As an example, if the gas source 10 is a self-inflating bag, the operator will decrease the degree of compression of the self-inflating bag for the next ventilation cycle to decrease the inspiratory volume delivered to the infant. As another example, if the gas source 10 is a flow-driven device, the operator may adjust one or more controls of the flow-driven device to decrease the inspiratory volume delivered to the infant for the next ventilation cycle.

Based on the leakage indicator 156a-c displayed on the display 150, the operator is able to observe how much of the inspiratory volume is being leaked to atmosphere for each ventilation cycle. If the leakage indicator 156b or 156c is displayed on the display 150, the operator may adjust the interface device 20 and/or any other aspect of the patient ventilation set-up 200 in order to reduce the amount of leakage for the next ventilation cycle. If the leakage indicator 156a is displayed on the display 150, the operator may continue with the current mode of operation with regard to leakage.

Accordingly, during resuscitation of an infant, an operator may be able to use the ventilation assistance device 100 to determine if the gas volume received by the infant for each ventilation cycle is appropriate for the weight/size of the infant. If the inspiratory volume is not appropriate for the weight/size of the infant being resuscitated, the operator will be able to adjust their mode of operating the gas source 10 so that the inspiratory volume delivered to the infant for the next berating cycle is more appropriate for the weight/size of the infant being resuscitated.

Further, during resuscitation of an infant, an operator may be able to use the ventilation assistance device 100 to determine the amount of leakage occurring during each ventilation cycle. If the amount of leakage is not within an acceptable range (e.g. within 0 - 30%), the operator may adjust the interface device 20 and/or any other aspect of the patient ventilation set-up 200 in order to reduce the amount of leakage for the next ventilation cycle.

Use of the ventilation assistance device 100 to train operators to resuscitate infants of different sizes as another non-limiting example will now be described.

The method of using the ventilation assistance device 100 to train operators to resuscitate infants is similar to the method described above for resuscitating infants, except that a manikin of an infant will be used to replicate ventilation of an infant. Accordingly, the interface device 20 will be positioned on the manikin instead of an infant. The manikin may be a manikin as described above or any other suitable manikin as understood in the art that is suitable for replicating ventilation of an infant.

During a resuscitation training exercise, an operator may desire to simulate resuscitating an infant having a particular weight/size. The operator operates the gas source 10 as described above to deliver an inspiratory volume to the manikin and to expel at least some of the inspiratory volume of gas subsequently exhaled by the manikin (i.e. the exhaled volume) for each ventilation cycle.

Similar to that described above, the operator will be able to observe if the gas volume received by the manikin is appropriate for the weight/size of the infant they simulating in the resuscitation training exercise. If the inspiratory volume is not appropriate for the weight/size of the infant they are simulating during the resuscitation exercise, the operator will adjust their mode of operating the gas source 10 as described above so that the inspiratory volume delivered to the manikin for the next ventilation cycle is more appropriate for the weight/size of the infant the operator is simulating in the resuscitation exercise.

As described above, the operator may also adjust the interface device 20 and/or any other aspect of the patient ventilation set-up 200 based on the leakage indicator 156a-c displayed on the display 150 during each ventilation cycle.

The operator can perform further resuscitation training exercises using the ventilation assistance device 100 to simulate resuscitating infants of different weights/sizes. The ventilation assistance device 100 may therefore be used to train operators to become more familiar with operating the gas source 10 for resuscitating infants of different weight/size. Accordingly, based on the resuscitation training exercises using the ventilation assistance device 100, an operator may be able to more effectively and consistently deliver the appropriate inspiratory volume for each ventilation cycle to infants of different weights/sizes.

As Figure 3 illustrates, ventilation assistance device 100 is positioned between the operator and the subject 30 with display 150 facing upwards, so that ready observation of the display 150 and the content viewable thereon is possible without the operator having to turn attention away from the subject 30. This continuous observation of subject 30, display 150 and interface device 20 avoids as far as possibly any interruption in or distraction from optimum operation of gas source 10, while providing constant feedback to the operator with regard to maintenance of that optimum operation by way of activation of the different indicators. It is noted that in example embodiments in which, audible, and/or haptic indications may be used, the orientation of the display may be less critical.

Further, the ventilation assistance device 100 may be used to train operators to correctly position the interface device 20 on an infant before commencing resuscitation of the infant in order to reduce leakage and how to adjust the interface device 20 and/or any other aspect of the patient ventilation set-up 200 in order to reduce the amount of leakage during each ventilation cycle.

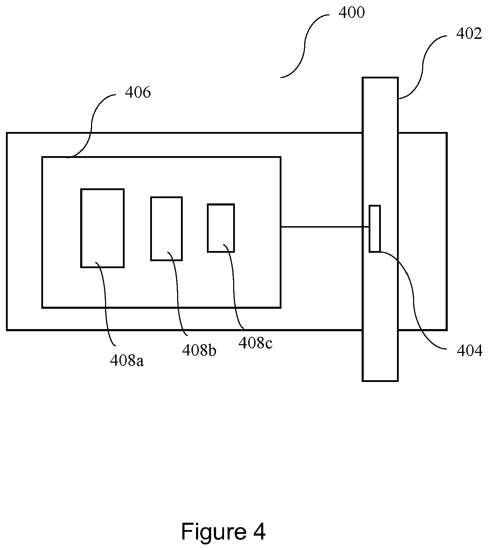

Figure 4 shows a schematic drawing illustrating a ventilation assistance device 400 according to an example embodiment, comprising a duct 402 configured to be connectable between a source of gas and an interface device, for establishing fluid communication between the source of gas and the interface device for delivering gas to a subject's airway; a flow sensor 404 configured to determine a flow volume of gas in the duct 402; and an operator interface 406 configured to represent a measure of gas received by the subject by way of a plurality of indicators 408a-c, each indicator 408a-c being representative of the measure being within a particular range suitable for respective reference subjects having respective characteristics; wherein the operator interface 406 is configured to display one of the plurality of indicators 408a-c based on the flow volume determined by the flow sensor 404 to represent to an operator the characteristic of the reference subject for which the gas received is suitable.

The indicators 408a-c may be respective images representing an observable physical characteristic of the respective reference subjects.

The respective characteristics may relate to the size of the respective reference subjects.

The measure of the flow volume is at least based on an expiratory volume flowing from the interface device through the duct as determined by the flow sensor 404. The measure of the flow volume may correspond to each expiratory volume determined by the flow sensor 404 from a continuous cycle of ventilation volumes.

The operator interface may be configured to provide a further representation of the flow volume, being a numerical representation.

The operator interface 406 may be configured to display a measure of leakage of gas from the interface device. The measure of leakage of gas may be determined based on an inspiratory volume also determined by the flow sensor 404. The measure of the flow volume may correspond to each inspiratory volume determined by the flow sensor 404 from a continuous cycle of ventilation volumes.

The measure of leakage of gas may be determined based on a difference between the inspiratory volume determined by the flow sensor and the expiratory volume determined by the flow sensor 404. The measure of the flow volume may correspond to each inspiratory volume and each corresponding expiratory volume determined by the flow sensor 404 from a continuous cycle of ventilation volumes.

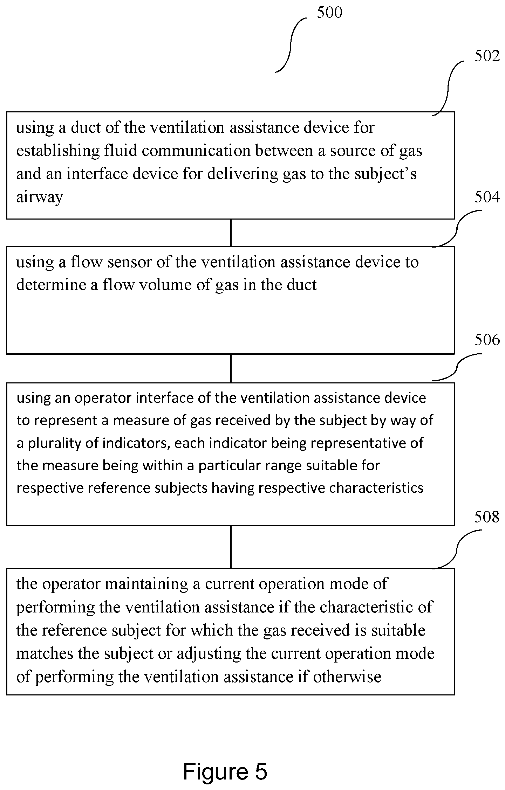

Figure 5 shows a flowchart 500 illustrating a method of using a ventilation assistance device for providing ventilation to a subject, according to an example embodiment. At step 502, a duct of the ventilation assistance device is used for establishing fluid communication between a source of gas and an interface device for delivering gas to the subject's airway. At step 504. a flow sensor of the ventilation assistance device is used to determine a flow volume of gas in the duct. At step 506, an operator interface of the ventilation assistance device is used to represent a measure of gas received by the subject by way of a plurality of indicators, each indicator being representative of the measure being within a particular range suitable for respective reference subjects having respective characteristics, wherein the operator interface is used to display one of the indicators based on the flow volume determined by the flow sensor to represent to an operator the characteristic of the reference subject for which the gas received is suitable. At step 508, the operator maintains a current operation mode of performing the ventilation assistance if the characteristic of the reference subject for which the gas received is suitable matches the subject or adjusting the current operation mode of performing the ventilation assistance if otherwise.

The indicators may be respective images representing an observable physical characteristic of the respective reference subjects.

The respective characteristics may relate to the size of the respective reference subjects.

The measure of the flow volume may be based on an expiratory volume flowing from the interface device through the duct as determined by the flow sensor.

The measure of the flow volume may correspond to each expiratory volume determined by the flow sensor from a continuous cycle of ventilation volumes.

The method may comprise using the operator interface to provide a further representation of the flow volume, being a numerical representation.

The method may comprise using the operator interface to display a measure of leakage of gas from the interface device. The measure of leakage of gas may be determined based on an inspiratory volume determined by the flow sensor. The measure of the flow volume corresponds to each inspiratory volume may be determined by the flow sensor from a continuous cycle of ventilation volumes.

The measure of leakage of gas may be determined based on a difference between the inspiratory volume determined by the flow sensor and the expiratory volume determined by the flow sensor. The measure of the flow volume may correspond to each inspiratory volume and each corresponding expiratory volume determined by the flow sensor from a continuous cycle of ventilation volumes.

The method may comprise the operator maintaining a current operation mode of performing the ventilation assistance if the measure of leakage is indicated as acceptable; or the operator adjusting the current operation mode of performing the ventilation assistance if otherwise.

The subject may be a human or a manikin of a human.

Aspects of the systems and methods described herein, such as, but not limited to, the display and its control, the flow sensor and its control, and the measurement and processing of the flow data, may be implemented as functionality programmed into any of a variety of circuitry, including programmable logic devices (PLDs), such as field programmable gate arrays (FPGAs), programmable array logic (PAL) devices, electrically programmable logic and memory devices and standard cell-based devices, as well as application specific integrated circuits (ASICs). Some other possibilities for implementing aspects of the system include: microcontrollers with memory (such as electronically erasable programmable read only memory (EEPROM)), embedded microprocessors, firmware, software, etc. Furthermore, aspects of the system may be embodied in microprocessors having software-based circuit emulation, discrete logic (sequential and combinatorial), custom devices, fuzzy (neural) logic, quantum devices, and hybrids of any of the above device types. Of course the underlying device technologies may be provided in a variety of component types, e.g., metal-oxide semiconductor field-effect transistor (MOSFET) technologies like complementary metal-oxide semiconductor (CMOS), bipolar technologies like emitter-coupled logic (ECL), polymer technologies (e.g., silicon-conjugated polymer and metal-conjugated polymer-metal structures), mixed analog and digital, etc.

The various functions or processes disclosed herein may be described as data and/or instructions embodied in various computer-readable media, in terms of their behavioral, register transfer, logic component, transistor, layout geometries, and/or other characteristics. Computer-readable media in which such formatted data and/or instructions may be embodied include, but are not limited to, non-volatile storage media in various forms (e.g., optical, magnetic or semiconductor storage media) and carrier waves that may be used to transfer such formatted data and/or instructions through wireless, optical, or wired signaling media or any combination thereof. When received into any of a variety of circuitry (e.g. a computer), such data and/or instruction may be processed by a processing entity (e.g., one or more processors).

The above description of illustrated embodiments of the systems and methods is not intended to be exhaustive or to limit the systems and methods to the precise forms disclosed. While specific embodiments of, and examples for, the systems components and methods are described herein for illustrative purposes, various equivalent modifications are possible within the scope of the systems, components and methods, as those skilled in the relevant art will recognize. The teachings of the systems and methods provided herein can be applied to other processing systems and methods, not only for the systems and methods described above.

It will be appreciated by a person skilled in the art that numerous variations and/or modifications may be made to the present invention as shown in the specific embodiments without departing from the scope of the invention as broadly described. The present embodiments are, therefore, to be considered in all respects to be illustrative and not restrictive. Also, the features described for different embodiments, including in the summary section, may be combined even if the feature or combination of features is not explicitly specified in the claims or the detailed description of the present embodiments, the invention being as defined in the appended claims.

In general, in the following claims, the terms used should not be construed to limit the systems and methods to the specific embodiments disclosed in the specification and the claims, but should be construed to include all processing systems that operate under the claims. Accordingly, the systems and methods are not limited by the disclosure, but instead the scope of the systems and methods is to be determined entirely by the claims.

Unless the context clearly requires otherwise, throughout the description and the claims, the words "comprise," "comprising," and the like are to be construed in an inclusive sense as opposed to an exclusive or exhaustive sense; that is to say, in a sense of "including, but not limited to." Words using the singular or plural number also include the plural or singular number respectively. Additionally, the words "herein," "hereunder," "above," "below," and words of similar import refer to this application as a whole and not to any particular portions of this application. When the word "or" is used in reference to a list of two or more items, that word covers all of the following interpretations of the word: any of the items in the list, all of the items in the list and any combination of the items in the list.

## Claims

1. A ventilation assistance device (100) comprising:
a duct (130) configured to be connectable between a source of gas (10) and an interface device (20), for establishing fluid communication between the source of gas (10) and the interface device (20) for delivering gas to a subject's airway;
a flow sensor (140) configured to measure a volume of gas flowing in the duct (130), the ventilation assistance device (100) being configured to calculate an expiratory volume of gas of the subject based on a measurement, by the flow sensor (130), of a volume of gas exhaled by the subject and flowing in the duct (130); and
an operator interface (150) configured to represent the calculated expiratory volume of gas by way of a plurality of indicators (154), each indicator (154) being representative of one of a plurality of reference subject groups having respective characteristics,
**characterised in that** the ventilation assistance device (100) is configured to:
determine, based on the calculated expiratory volume of gas, for which of the plurality of reference subject groups the calculated expiratory volume of gas is suitable; and
display, on the operator interface, one of the plurality of indicators (154) to represent to an operator the characteristic of the reference subject group for which the calculated expiratory volume of gas has been determined to be suitable.

2. The device of claim 1, wherein the indicators (154) are respective images representing an observable physical characteristic of the respective reference subject groups.

3. The device of claim 1 or claim 2, wherein the respective characteristics relate to the size of the respective reference subject groups.

4. The device of any one of claims 1 to 3, wherein the calculated expiratory volume of gas corresponds to each expiratory volume determined by the flow sensor (140) from a continuous cycle of ventilation volumes.

5. The device of any one of claims 1 to 4, wherein the operator interface (150) is configured to provide a further representation of the calculated expiratory volume of gas, being a numerical representation (152).

6. The device of any one of the claims 1 to 5, wherein the operator interface (150) is configured to display a measure of leakage of gas (156) from the interface device (20).

7. The device of claim 6, wherein the measure of leakage of gas (156) is determined based on an inspiratory volume determined by the flow sensor (140).

8. A method of using a ventilation assistance device (100), the method comprising the steps of:
using a duct (130) of the ventilation assistance device (100) for establishing fluid communication between a source of gas (10) and an interface device (20);
using a flow sensor (140) of the ventilation assistance device (100) to calculate an expiratory volume of gas of a
subject based on a measurement, by the flow sensor (130), of a volume of gas exhaled by the subject and flowing in the duct (130); and
using an operator interface (150) of the ventilation assistance device (100) to represent the calculated expiratory volume of gas by way of a plurality of indicators (154), each indicator (154) being representative one of a plurality of reference subject groups having respective characteristics,
**characterised by** the following steps:
determining, based on the calculated expiratory volume of gas, for which one of the plurality of reference subject groups the calculated expiratory volume of gas is suitable; and
displaying, on the operator interface (150), one of the plurality of indicators (154) to represent to an operator the characteristic of the reference subject group for which the calculated expiratory volume of gas has been determined to be suitable.

9. The method of claim 8, wherein the indicators (154) are respective images representing an observable physical characteristic of the respective reference subject groups.

10. The method of claim 8 or claim 9, wherein the respective characteristics relate to the size of the respective reference subject groups.

11. The method of any one of claims 8 to 10, wherein the calculated expiratory volume of gas corresponds to each expiratory volume determined by the flow sensor (140) from a continuous cycle of ventilation volumes.

12. The method of any one of claims 8 to 11, comprising using the operator interface (150) to provide a further representation of the calculated expiratory volume of gas, being a numerical representation (152).

13. The method of any one of the claims 8 to 12, comprising using the operator interface to display a measure of leakage of gas (156) from the interface device (20).

14. The method of claim 13, comprising using the flow sensor (140) to determine the measure of leakage of gas (156) based on an inspiratory volume determined by the flow sensor (140).

15. The method of any one of claims 8 to 14, wherein the subject is a human or a manikin of a human.

## Patentansprüche

1. Eine Beatmungshilfe Vorrichtung (100) aufweisend:
einem Kanal (130), der zwischen einer Gasquelle (10) und einer Schnittstellenvorrichtung (20) verbunden werden kann, um eine Fluidverbindung zwischen der Gasquelle (10) und der Schnittstellenvorrichtung (20) herzustellen und Gas in die Atemwege eines Probanden zu leiten;
einem Durchflusssensor (140), der das im Kanal (130) strömende Gasvolumen misst, wobei die Beatmungshilfe Vorrichtung (100) konfiguriert ist, auf Grundlage der Messung, durch den Durchflusssensor (130), des vom Probanden ausgeatmeten und im Kanal (130) strömenden Gasvolumens das Ausatemvolumen des Probanden zu berechnen; und
eine Bedienerschnittstelle (150), die so konfiguriert ist, dass sie das berechnete Ausatemvolumen des Gases mittels mehrerer Indikatoren (154) darstellt, wobei jeder Indikator (154) eine von mehreren Referenzpersonengruppen mit jeweiligen Eigenschaften repräsentiert,
**gekennzeichnet dadurch, dass** das Beatmungshilfe Vorrichtung (100) konfiguriert ist:
auf Grundlage des berechneten Ausatemvolumens des Gases zu bestimmen, für welche der mehreren Referenzpersonengruppen das berechnete Ausatemvolumen des Gases geeignet ist; und
auf der Bedienerschnittstelle einen der Indikatoren (154) anzuzeigen, um dem Bediener die Eigenschaft der Referenzpersonengruppe zu verdeutlichen, für die das berechnete Ausatemvolumen des Gases als geeignet ermittelt wurde.

2. Die Vorrichtung gemäß Anspruch 1, wobei es sich bei den Indikatoren (154) um Bilder handelt, die jeweils eine beobachtbare physikalische Eigenschaft der jeweiligen Referenzsubjektgruppen darstellen.

3. Die Vorrichtung gemäß Anspruch 1 oder Anspruch 2, wobei sich die jeweiligen Merkmale auf die Größe der jeweiligen Referenzsubjektgruppen beziehen.

4. Die Vorrichtung gemäß einem der Ansprüche 1 bis 3, wobei das berechnete Ausatemvolumen des Gases jedem Ausatemvolumen entspricht, das vom Durchflusssensor (140) aus einem kontinuierlichen Zyklus von Beatmungsvolumina ermittelt wird.

5. Die Vorrichtung gemäß einem der Ansprüche 1 bis 4, wobei die Bedienerschnittstelle (150) so konfiguriert ist, dass sie eine weitere Darstellung des berechneten Ausatemvolumens von Gas, nämlich eine numerische Darstellung (152), bereitstellt.

6. Die Vorrichtung gemäß einem der Ansprüche 1 bis 5, wobei die Bedienerschnittstelle (150) so konfiguriert ist, dass sie ein Maß für Gasaustritt (156) aus der Schnittstellenvorrichtung (20) anzeigt.

7. Die Vorrichtung gemäß Anspruch 6, wobei das Maß für den Gasaustritt (156) auf der Grundlage eines Mittels des vom Durchflusssensor (140) ermittelten Inspirationsvolumens bestimmt wird.

8. Ein Verfahren zur Verwendung einer Beatmungshilfe Vorrichtung (100), wobei das Verfahren folgende Schritte aufweist:
Verwendung eines Kanals (130) der Beatmungshilfe Vorrichtung (100) zur Herstellung einer Fluidverbindung zwischen einer Gasquelle (10) und einer Schnittstelle (20);
Verwendung eines Durchflusssensors (140) der Beatmungshilfe Vorrichtung (100) zur Berechnung des Ausatemvolumens eines Probanden auf Grundlage der Messung, durch den Durchflusssensors (130), des vom Probanden ausgeatmeten und im Kanal (130) strömenden Gasvolumens; und
Verwendung einer Bedienerschnittstelle (150) der Beatmungshilfe Vorrichtung (100) zur Darstellung des berechneten Ausatemvolumens mittels mehrerer Indikatoren (154), wobei jeder Indikator (154) eine von mehreren Referenzprobandengruppen mit spezifischen Eigenschaften repräsentiert,
durch folgende Schritte gekennzeichnet:
Bestimmen, basierend auf dem berechneten Ausatemvolumen, für welche der mehreren Referenzprobandengruppen das berechnete Ausatemvolumen geeignet ist; und
Anzeigen, auf der Bedienerschnittstelle (150), einen der Indikatoren (154), um dem Bediener die Charakteristik der Referenzpersonengruppe zu verdeutlichen, für die das berechnete Ausatemvolumen an Gas als geeignet bestimmt wurde.

9. Verfahren gemäß Anspruch 8, wobei es sich bei den Indikatoren (154) um Bilder handelt, die jeweils eine beobachtbare physikalische Eigenschaft der jeweiligen Referenzsubjektgruppen darstellen.

10. Verfahren gemäß Anspruch 8 oder 9, wobei sich die jeweiligen Merkmale auf die Größe der jeweiligen Referenzsubjektgruppen beziehen.

11. Verfahren gemäß einem der Ansprüche 8 bis 10, wobei das berechnete Ausatemvolumen des Gases jedem Ausatemvolumen entspricht, das vom Durchflusssensor (140) aus einem kontinuierlichen Zyklus von Beatmungsvolumina ermittelt wird.

12. Verfahren gemäß einem der Ansprüche 8 bis 11, aufweisend die Verwendung der Bedienerschnittstelle (150) zum Bereitstellen einer weiteren Darstellung des berechneten Ausatemvolumens von Gas, wobei es sich um eine numerische Darstellung (152) handelt.

13. Verfahren gemäß einem der Ansprüche 8 bis 12, aufweisend die Verwendung der Bedienerschnittstelle zur Anzeige eines Messwerts für Gasaustritt (156) aus der Schnittstellenvorrichtung (20).

14. Verfahren gemäß Anspruch 13, aufweisend die Verwendung des Durchflusssensors (140) zur Bestimmung des Gasaustritts (156) auf der Grundlage eines mit dem Durchflusssensor (140) ermittelten Inspirationsvolumens.

15. Verfahren gemäß einem der Ansprüche 8 bis 14, wobei es sich bei dem Probanden um einen Menschen oder eine Nachbildung eines Menschen handelt.

## Revendications

1. Dispositif d'assistance à la ventilation (100), comprenant :
un conduit (130) configuré pour être connectable entre une source de gaz (10) et un dispositif d'interface (20), afin d'établir une communication fluidique entre la source de gaz (10) et le dispositif d'interface (20) pour délivrer du gaz aux voies respiratoires d'un sujet,
un capteur de débit (140) configuré pour mesurer un volume de gaz s'écoulant dans le conduit (130), le dispositif d'assistance à la ventilation (100) étant configuré pour calculer un volume expiratoire de gaz du sujet sur la base d'une mesure, par le capteur de débit (130), d'un volume de gaz expiré par le sujet et s'écoulant dans le conduit (130) ; et
une interface opérateur (150) configurée pour représenter le volume expiratoire de gaz calculé au moyen d'une pluralité d'indicateurs (154), chaque indicateur (154) étant représentatif d'un groupe parmi une pluralité de groupes de sujets de référence ayant des caractéristiques respectives,
**caractérisé en ce que** le dispositif d'assistance à la ventilation (100) est configuré pour :
déterminer, sur la base du volume expiratoire calculé de gaz, pour lequel parmi la pluralité de groupes de sujets de référence le volume expiratoire calculé est approprié ; et
afficher, sur l'interface opérateur, l'un de la pluralité d'indicateurs (154) pour représenter à un opérateur la caractéristique du groupe de sujets de référence pour lequel le volume expiratoire calculé de gaz a été déterminé comme étant approprié.

2. Dispositif selon la revendication 1, dans lequel les indicateurs (154) sont des images respectives représentant une caractéristique physique observable des groupes de sujets de référence respectifs.

3. Dispositif selon la revendication 1 ou la revendication 2, dans lequel les caractéristiques respectives se rapportent à la taille des groupes de sujets de référence respectifs.

4. Dispositif selon l'une quelconque des revendications 1 à 3, dans lequel le volume expiratoire calculé de gaz correspond à chaque volume expiratoire déterminé par le capteur de débit (140) à partir d'un cycle continu de volumes de ventilation.

5. Dispositif selon l'une quelconque des revendications 1 à 4, dans lequel l'interface opérateur (150) est configurée pour fournir une représentation supplémentaire du volume expiratoire calculé de gaz, étant une représentation numérique (152).

6. Dispositif selon l'une quelconque des revendications 1 à 5, dans lequel l'interface opérateur (150) est configurée pour afficher une mesure de fuite de gaz (156) provenant du dispositif d'interface (20).

7. Dispositif selon la revendication 6, dans lequel la mesure de fuite de gaz (156) est déterminée sur la base d'un volume inspiratoire déterminé par le capteur de débit (140).

8. Procédé d'utilisation d'un dispositif d'assistance à la ventilation (100), le procédé comprenant les étapes suivantes consistant à :
utiliser un conduit (130) du dispositif d'assistance à la ventilation (100) pour établir une communication fluidique entre une source de gaz (10) et un dispositif d'interface (20) ;
utiliser un capteur de débit (140) du dispositif d'assistance à la ventilation (100) pour calculer un volume expiratoire de gaz d'un sujet sur la base d'une mesure, par le capteur de débit (130), d'un volume de gaz expiré par le sujet et s'écoulant dans le conduit (130) ; et
utiliser une interface opérateur (150) du dispositif d'assistance à la ventilation (100) pour représenter le volume expiratoire calculé de gaz au moyen d'une pluralité d'indicateurs (154), chaque indicateur (154) étant représentatif d'un groupe parmi une pluralité de groupes de sujets de référence ayant des caractéristiques respectives,
**caractérisé par** les étapes suivantes consistant à :
déterminer, sur la base du volume expiratoire calculé de gaz, pour lequel parmi la pluralité de groupes de sujets de référence le volume expiratoire calculé de gaz est approprié ; et
afficher, sur l'interface opérateur (150), l'un parmi la pluralité d'indicateurs (154) pour représenter à un opérateur la caractéristique du groupe de sujets de référence pour lequel le volume expiratoire calculé de gaz a été déterminé comme étant approprié.

9. Procédé selon la revendication 8, dans lequel les indicateurs (154) sont des images respectives représentant une caractéristique physique observable des groupes de sujets de référence respectifs.

10. Procédé selon la revendication 8 ou la revendication 9, dans lequel les caractéristiques respectives se rapportent à la taille des groupes de sujets de référence respectifs.

11. Procédé selon l'une quelconque des revendications 8 à 10, dans lequel le volume expiratoire calculé de gaz correspond à chaque volume expiratoire déterminé par le capteur de débit (140) à partir d'un cycle continu de volumes de ventilation.

12. Procédé selon l'une quelconque des revendications 8 à 11, comprenant l'utilisation de l'interface opérateur (150) pour fournir une représentation supplémentaire du volume expiratoire calculé de gaz, étant une représentation numérique (152).

13. Procédé selon l'une quelconque des revendications 8 à 12, comprenant l'utilisation de l'interface opérateur pour afficher une mesure de fuite de gaz (156) provenant du dispositif d'interface (20).

14. Procédé selon la revendication 13, comprenant l'utilisation du capteur de débit (140) pour déterminer la mesure de fuite de gaz (156) sur la base d'un volume inspiratoire déterminé par le capteur de débit (140).

15. Procédé selon l'une quelconque des revendications 8 à 14, dans lequel le sujet est un être humain ou un mannequin représentant un être humain.
